# EUROPEAN PATENT APPLICATION

(11) **EP 4 616 891 A1**
(43) Date of publication of application: **17.09.2025**
(21) Application number: 23887546.2
(22) Date of filing: 03.08.2023
(51) Int. Cl.: A61M 15/00

(54) **COUNTING MECHANISM AND INHALATION DEVICE**

(30) Priority: 09.11.2022 CN 202222990576 U
(71) Applicant: Transpire Bio Inc, Weston, FL 33331 (US)
(72) Inventor: OUYANG, Guanglin, Shenzhen, Guangdong 518102 (CN); ZHANG, Xieen, Shenzhen, Guangdong 518102 (CN)
(74) Representative: Latscha Schöllhorn Partner AG
(86) International application number: PCT/CN2023/110916
(87) International publication number: WO 2024/098850

(57) **Abstract**

This application relates to a counting mechanism and an inhalation device. The counting mechanism comprises: a shell, including a housing and a magnifying lens, where the housing has an observation window, and the magnifying lens is mounted on the housing and blocks the observation window; and a counting assembly, positioned inside the housing and configured to form different counts at the observation window. The counting mechanism can be used in an inhalation device for atomizing an aerosol-forming material. Each time the inhalation device is used, a counting assembly counts once, to display the number of times of atomization or display the remaining number of times of atomization, so that a user can clearly learn the usage of the aerosol-forming material to replace a new tank or inhalation device in a timely manner. **In** addition, the magnifying lens is positioned at the observation window of the counting mechanism for displaying the count, and the count formed by the counting assembly can be magnified by the magnifying lens, so that the count can be clearly displayed. Moreover, the counting mechanism does not need to be set to a large volume to mark a large number, thereby saving an internal space of the housing.

## Description

### TECHNICAL FIELD

This application relates to the field of atomization technologies, and in particular, to a counting mechanism and an inhalation device.

### BACKGROUND

In the field of medical care technologies, an aerosol-forming material is generally atomized into an aerosol by an inhalation device for a patient to inhale. For a conventional medical inhalation device, the aerosol-forming material is generally a liquid medicine or powder. However, in a process of the liquid medicine or powder being sprayed out from the inhalation device and continuously consumed, a user cannot accurately ascertain what amount of the medicine or powder that has been used, which makes it difficult to determine when a new liquid tank, cartridge, or inhalation device need to be replaced.

### SUMMARY

According to certain aspects, a counting mechanism and an inhalation device are described.

In an embodiment, a counting mechanism is provided that is configured to count the number of atomizations performed by an inhalation device. The counting mechanism comprises a shell including a housing and a magnifying lens, where the housing has an observation window and the magnifying lens is mounted at the observation window; and a counting assembly positioned inside the housing and configured to form different counts at the observation window.

In an embodiment, the magnification of the magnifying lens is 2x to 3x.

In an embodiment, the counting assembly comprises a first driving disk and a first counting disk that are rotatably positioned on the housing, and for
each time the first driving disk rotates, the first counting disk rotates along with the rotation of the first driving disk, and the count changes once.

In an embodiment, when the first counting disk rotates to a rotation limit position, the first counting disk abuts against and is limited by the housing.

In an embodiment, a reminder mark is provided on the first counting disk, and when the first counting disk rotates to the rotation limit position, the reminder mark rotates to the observation window.

In an embodiment, the counting assembly comprises a second driving disk, a second counting disk, and a third counting disk that are rotatably positioned on the housing, the second counting disk has a geared connection with the second driving disk, and the third counting disk has a geared connection with the second counting disk, where
each time the second driving disk rotates, the second counting disk rotates along with the rotation of the second driving disk, and a number on the second driving disk change once; and a number on the third counting disk changes once when the second counting disk rotates to a preset position, and the number on the second counting disk and the number on the third counting disk form the count together.

In an embodiment, a receiving space is provided on the second counting disk, the third counting disk is eccentrically sheathed in the receiving space, and any number on the third counting disk and any number on the second counting disk are rotated to be adjacent and to form the count together.

In an embodiment, the second counting disk comprises a second disk body and a ring gear, a plurality of numbers are provided on the second disk body, and the ring gear is positioned on the second disk body and has a geared connection with the second driving disk.

In an embodiment, transmission teeth are circumferentially positioned on an inner periphery of the ring gear, and the second driving disk is positioned in the ring gear and is in mesh transmission with the transmission teeth.

In an embodiment, the counting mechanism further comprises a transmission gear, the transmission gear has a geared connection between the ring gear and the third counting disk, and the third counting disk is driven by the transmission gear to rotate and undergo a number change only when the second counting disk rotates to the preset position.

In an embodiment, an activator tooth is positioned on an outer periphery of the ring gear, and the activator tooth is in mesh transmission with the transmission gear only when the second counting disk rotates to the preset position.

An inhalation device comprises an atomization assembly and the counting mechanism. Each time the atomization assembly performs atomization, the count is coupled and changes once.

The counting mechanism can be used in an inhalation device for atomizing an aerosol-forming material. Each time the inhalation device is used, a counting assembly counts once, to display the number of times of atomization or display the remaining number of times of atomization, so that a user can clearly learn the usage of the aerosol-forming material to replace a new tank or inhalation device in a timely manner. In addition, the magnifying lens is positioned at the observation window of the counting mechanism for displaying the count, and the count formed by the counting assembly can be magnified by the magnifying lens, so that the count can be clearly displayed. Moreover, the counting mechanism does not need to be set to a large volume to mark a large number, thereby saving an internal space of the housing.

### BRIEF DESCRIPTION OF THE DRAWINGS

To describe the technical solutions in embodiments of this application more clearly, the following briefly introduces the accompanying drawings describing the embodiments or the related art. The accompanying drawings show merely some embodiments of this application, and a person of ordinary skill in the art would understand that variations from the accompanying drawings can be made within the scope of this disclosure.
FIG. 1 is an exploded view of a counting mechanism according to an embodiment of the utility model;
FIG. 2 is a cross-sectional view of the counting mechanism shown in FIG. 1;
FIG. 3 is a perspective view of the counting mechanism shown in FIG. 1;
FIG. 4 is a perspective view of a housing in the counting mechanism shown in FIG. 1;
FIG. 5 is an exploded view of a counting mechanism according to another embodiment of the utility model;
FIG. 6 is a schematic diagram of an interior of the counting mechanism shown in FIG. 5;
FIG. 7 is a top view of a second counting disk in the counting mechanism shown in FIG. 6;
FIG. 8 is a top view of a third counting disk in the counting mechanism shown in FIG. 6; and
FIG. 9 is a cross-sectional view of the counting mechanism shown in FIG. 6.

Descriptions of reference numbers: 100. counting mechanism; 10. shell; 12. observation window; 14. first protrusion; 20. counting assembly; 22. first driving disk; 24. first counting disk; 241. reminder mark; 243. second protrusion; 30. second driving disk; 40. magnifying lens; 50. second counting disk; 51. receiving space; 52. second disk body; 54. ring gear; 541. transmission tooth; 542. first limiting groove; 543. activator tooth; 60. first limiting member; 70. third counting disk; 72. third disk body; 74. gear portion; 741. second limiting groove; 80. transmission gear; and 90. second limiting member.

### DETAILED DESCRIPTION

To make the foregoing objects, features, and advantages of this application clearer and easier to understand, the following further describes specific implementations of this application with reference to the accompanying drawings. In the following descriptions, many specific details are described for ease of full understanding of this application. However, this application can be implemented in many other manners different from those described herein. A person skilled in the art can make similar improvements without departing from the scope of this application. Therefore, this application is not limited to the specific embodiments disclosed below.

In the descriptions of this application, it should be understood that orientation or position relationships indicated by terms such as "center", "longitudinal", "transverse", "length", "width", "thickness", "up", "down", "front", "rear", "left", "right", "vertical", "horizontal", "top", "bottom", "inside", "outside", "clockwise", "anticlockwise", "axial direction", "radial direction", and "circumferential direction" are based on orientation or positional relationships shown in the drawings, and are merely used for ease and brevity of description of this application, rather than indicating or implying that the mentioned device or element needs to have a particular orientation or be constructed and operated in a particular orientation. Therefore, such terms should not be construed as a limitation on this application unless expressly stated otherwise.

In addition, the terms "first" and "second" are merely used for the purpose of description, and should not be construed as indicating or implying relative importance or implicitly indicating a quantity of the indicated technical features. Therefore, features defined by "first" or "second" can explicitly or implicitly include at least one of such features. In the descriptions of this application, unless otherwise explicitly defined, "a plurality of" means at least two, such as two or three.

In this application, unless otherwise explicitly specified or defined, terms such as "mount", "connect", "connection", and "fix" should be understood in a broad sense. For example, the connection can be a fixed connection, a detachable connection, or an integral connection; the connection can be a mechanical connection or an electrical connection; or the connection can be a direct connection, an indirect connection through an intermediary, internal communication between two elements, or interaction between two elements, unless otherwise explicitly defined. A person of ordinary skill in the art would understand the specific meanings of the foregoing terms in this application according to specific situations.

In this application, unless otherwise explicitly specified and defined, a first feature being "on" or "under" a second feature can mean that the first feature is in direct contact with the second feature, or the first feature is in indirect contact with the second feature by using an intermediate element. In addition, the first feature being described as "above", "over", or "on" the second feature means that the first feature can be directly or obliquely above the second feature, or merely indicates that the first feature is at a higher horizontal position than the second feature. The first feature being "below", "under", and "beneath" the second feature can mean that the first feature is directly or obliquely below the second feature, or merely indicate that the first feature is at a lower horizontal position than the second feature.

It should be noted that when an element is referred to as "being fixed to" or "being positioned on" another element, the element can be directly located on the another element, or an intermediate element can exist therebetween. When an element is considered to be "connected to" another element, the element can be directly connected to the another element, or an intermediate element can also exist therebetween. The terms "vertical", "horizontal", "up", "down", "left", "right", and similar expressions used in this specification are merely used for illustration and do not indicate a unique implementation.

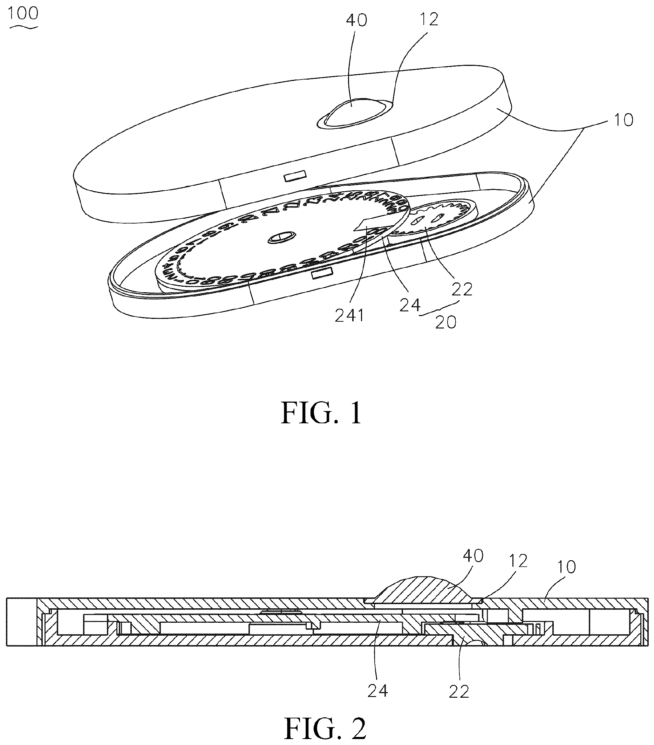

Referring to FIG. 1 and FIG. 2, in an embodiment of this application, a counting mechanism 100 is provided, which is configured to count the number of times an inhalation device is actuated to dispense a dose of medicine. Counting mechanism 100 comprises a shell 10 and a counting assembly 20. The shell 10 comprises a housing and a magnifying lens 40. The housing has an observation window 12, and the magnifying lens 40 is mounted over the observation window 12. The counting assembly 20 is positioned inside the housing and is configured to form different numbers at the observation window 12. In some instances, the counting mechanism 100 can be used in an inhalation device that atomizes an aerosol-forming material. Each time the inhalation device is used, the counting assembly 20 counts once, to display the number of times atomization/actuation has occurred or display a remaining number of atomizations/actuations remaining. A user can clearly monitor through the observation window the usage of the aerosol-forming material, and thus know when to replace a used medicine-containing cartridge or the inhalation device itself in a timely manner.

In some embodiments, magnifying lens 40 is positioned over observation window 12 of the counting mechanism 100 that displays a count, and the count formed by the counting assembly 20 can be magnified by the magnifying lens 40 so that the count can be clearly observed. Moreover, the counting mechanism 100 does not need to be set to display a wide range of numbers to mark a high number, thereby saving an internal space of the housing.

In some embodiments, the magnification of the magnifying lens 40 is 2x to 3x. For example, a number height and a number width on the counting assembly 20 can be 2.3 mm and 2.5 mm, respectively, and a number height and a number width after magnification are 6.0 mm and 6.5 mm, respectively. **In** some cases, the magnification is 2.5x. **In** an embodiment, a material of the magnifying lens 40 is polycarbonate ("PC"), the thickness of the magnifying lens 40 is 2.5 mm, and the object distance between the magnifying lens 40 and text is 1.5 mm.

**In** some embodiments, the magnifying lens 40 can be one of a convex lens or a Fresnel lens.

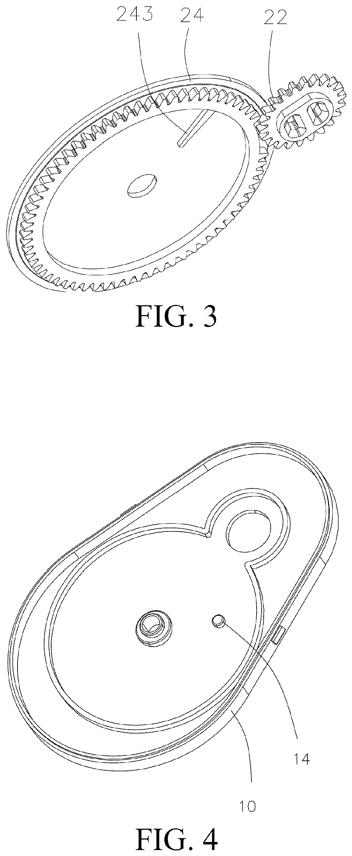

Referring to FIG. 1 to FIG. 4, in some embodiments, the counting assembly 20 comprises a first driving disk 22 and a first counting disk 24 that are rotatably positioned on the housing. Each time the first driving disk 22 rotates, the first counting disk 24 rotates along with the rotation of the first driving disk 22, and the count changes once. Thus, when the inhalation device is used once, the first driving disk 22 is driven to rotate once, so that the first counting disk 24 counts once. In this way, the number of uses of the inhalation device can be counted.

A surface of the first counting disk 24 facing the magnifying lens 40 is circumferentially provided with a plurality of numbers at intervals. Any number can rotate to the observation window 12 with rotation of the first counting disk 24, and form the count together. Thus, a plurality of numbers are marked on the surface of the first counting disk 24 facing the magnifying lens 40. When the first counting disk 24 rotates, different numbers can be rotated to the observation window 12 at the magnifying lens 40 so that the count is displayed at the observation window 12 and magnified by the magnifying lens 40.

In some embodiments, when the first counting disk 24 rotates to a rotation limit position, the first counting disk 24 abuts against and is limited by the housing. Stated differently, when the count reaches a maximum or minimum limit value, the first counting disk 24 is limited at the rotation limit position, and no longer rotates or changes the displayed count. Using an example where the numbers marked on the first counting disk 24 are 0 to 30, and the counting mechanism 100 is configured to display a remaining number atomizations/actuations left in an inhaler, the count displayed in an initial state is 30. After each atomization or actuation of the inhaler, the count gradually decreases towards 0, a number between 0 and 30 on the first counting disk 24 is rotated to the observation window 12. When 0 is eventually reached, the 0 indicates that the remaining number of atomizations or actuations is zero, and the inhalation device cannot be used any longer. In addition, when at 0 on the counting assembly 20, the first counting disk 24 is also limited at the rotation limit position, and cannot rotate any more, to effectively remind a user that the inhalation device is no longer operable and does not contain any remaining doses of medicine.

In some embodiments, a reminder mark 241 is provided on the first counting disk 24. When the first counting disk 24 rotates to the rotation limit position, the reminder mark 241 rotates to the observation window 12. For example, the reminder mark 241 is provided between 0 and 30. After the count gradually decreases from 30 to 0, the reminder mark 241 rotates to the observation window 12, to remind the user that the remaining number of atomizations or actuations is zero, and the inhalation device cannot be used any more.

In some cases, a first protrusion 14 is positioned on the housing, and a second protrusion 243 is positioned on the first counting disk 24. When the first counting disk 24 rotates to the rotation limit position, the first protrusion 14 abuts against and is prevented from further rotation by the second protrusion 243.

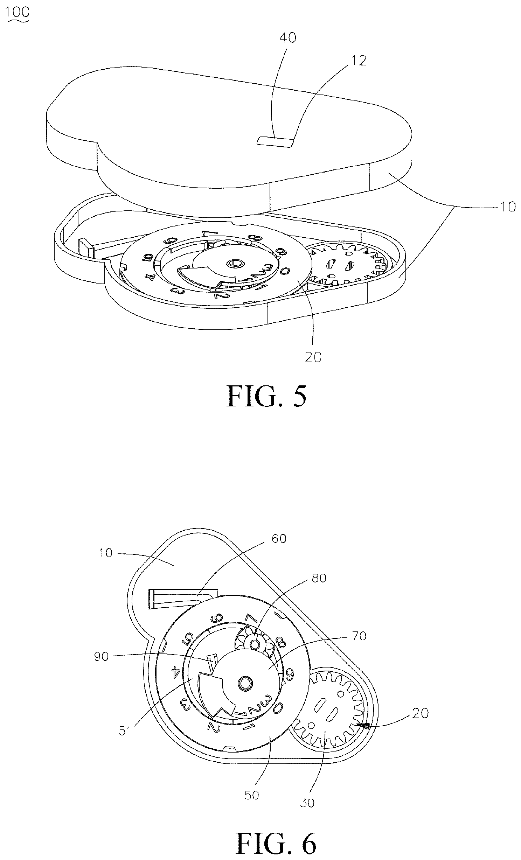

Referring to FIG. 5 and FIG. 6, in some embodiments, the counting assembly 20 comprises a second driving disk 30, a second counting disk 50, and a third counting disk 70. The second driving disk 30, the second counting disk 50, and the third counting disk 70 are rotatably positioned on the shell 10, the second counting disk 50 has a geared connection with the second driving disk 30, and the third counting disk 70 has a geared connection with the second counting disk 50. Each time the second driving disk 30 rotates, the second counting disk 50 rotates along with the rotation of the second driving disk 30, and a number on the second counting disk changes once. A number on the third counting disk 70 changes once only when the second counting disk 50 rotates to a preset position. The number on the second counting disk 50 and the number on the third counting disk 70 form a count together.

The counting mechanism 100 can be used in an inhalation device for atomizing an aerosol-forming material. Each time the inhalation device is used, the second driving disk 30 can be driven to rotate once, so that the second counting disk 50 and the third counting disk 70 rotate along with the second driving disk 30. The number on the second counting disk 50 and the number on the third counting disk 70 form a count together. Specifically, when the inhalation device performs an atomization or actuation, and the second driving disk 30 drives the second counting disk 50 to rotate once to change a number one numerical value. An atomization process of the inhalation device usually requires actuation and cooperation of an internal driving mechanism (not shown in the figure, but known in the art). In some embodiments, a known internal driving mechanism is mechanically coupled to the second driving disk 30. During the actuation of the internal driving mechanism, the second driving disk 30 is synchronously driven to be actuated, thereby completing processes of delivering medicine and counting at the same time.

In addition, the third counting disk 70 synchronously rotates along with an atomization assembly together with the second counting disk 50 once, but only when the second counting disk 50 rotates to a preset position, with each atomization or actuation of the inhalation device resulting in a single number change on the third counting disk 70. As shown in FIG. 6, this movement occurs when the number of counts by the second counting disk 50 exceeds ten, causing the third counting disk 70 to rotate once to represent the tens digit of the number of times. In this way, the number on the second counting disk 50 and the number on the third counting disk 70 form a count together, displaying the number of times atomization or actuation have been performed by the inhalation device, or the number atomization or actuation remaining in the inhalation device. A user can accordingly determine the amount of aerosol-forming material in the inhalation device, and understand when replacement of a cartridge or inhalation device is needed.

Further, a receiving space 51 is provided on the second counting disk 50, the third counting disk 70 is eccentrically positioned in the receiving space 51, and any number on the third counting disk 70 and any number on the second counting disk 50 can be rotated to be adjacent and to form the count together. In this way, the third counting disk 70 is eccentrically received in the receiving space 51 of the second counting disk 50, so that any number on the third counting disk 70 and any number on the second counting disk 50 can be adjacent and form the count together.

In some embodiments, the number change on the second counting disk 50 is a change of the ones digit, and the digit change on the third counting disk 70 is a change of the tens digit. In this way, through cooperation between the second counting disk 50 and the third counting disk 70, different numbers on the second counting disk 50 and different numbers on the third counting disk 70 form the count together, so that the number of times of atomization that can be counted can reach a number having a tens digit.

Specifically, the second counting disk 50 changes the number once by rotating by a first preset degree, and the third counting disk 70 changes the number once by rotating by a second preset degree. In other words, for the second counting disk 50 on which the unit digit changes, a complete circle is divided into ten parts, numbers on the second counting disk 50 are 0 to 9, and each time the second counting disk 50 rotates by 36 degrees (namely, the first preset degree), the number changes once, for example, the number changes from 0 to 9, or changes from 9 to 8.

In addition, the numbers on the third counting disk 70 can be 0 to 3, 0 to 6, or the like, which is not limited herein. In addition, the number 0 on the third counting disk 70 can be marked by using different mark that indicates replacement, for example, a red mark, to provide an eye-catching reminder when the amount of aerosol-forming material becomes zero.

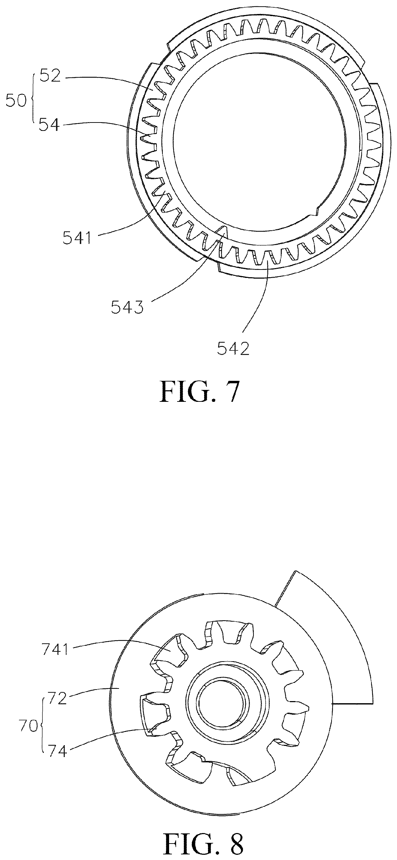

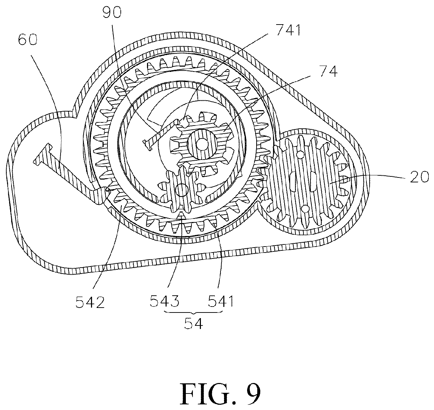

Referring to FIG. 6, FIG. 7, and FIG. 9, in some embodiments, the second counting disk 50 comprises a second disk body 52 and a ring gear 54. The second disk body 52 is provided with a plurality of numbers on a surface. The ring gear 54 is positioned on the second disk body 52 and has a geared connection with the second driving disk 30. In this way, through the transmission connection between the ring gear 54 and the second driving disk 30, the second driving disk 30 drives the second disk body 52 to rotate when rotating, so that the number viewable through observation window 12 on the second disk body 52 changes.

Further, transmission teeth 541 are circumferentially positioned on an inner periphery of the ring gear 54. The second driving disk 30 is mechanically connected in the ring gear 54 via a mesh transmission with the transmission teeth 541. In this way, the second driving disk 30 overlaps the ring gear 54 to save a mounting space, and can implement a transmission connection between the second driving disk 30 and the ring gear 54.

In some embodiments, the counting mechanism 100 further comprises a transmission gear 80 in a geared connection between the ring gear 54 and the third counting disk 70, and the third counting disk 70 is driven by the transmission gear 80 to rotate and undergo a number change only when the second counting disk 50 rotates to the preset position. In this way, the second counting disk 50 has a geared connection with the third counting disk 70 by using the transmission gear 80, so that the third counting disk 70 rotates once when the second counting disk 50 rotates to the preset position.

Further, an activator tooth 543 is positioned on an inner periphery of the ring gear 54, and the activator tooth 543 is in mesh transmission with the transmission gear 80 only when the second counting disk 50 rotates to a preset position. In some embodiments, one or two activator teeth 543 are positioned on the inner periphery of the ring gear 54 of the second counting disk 50. In this way, during rotation of the second counting disk 50, the activator tooth 543 is intermittently meshed with and transmitted to the transmission gear 80, so that the third counting disk 70 performs an intermittent movement driven by the transmission gear 80. A second ring gear 54 does not need to rotate all the time with a first ring gear 54, and the number on the second ring gear 54 changes once when the first ring gear 54 rotates to the preset position.

In this embodiment, the counting mechanism 100 is configured to display the remaining number of use times. The numbers on the second counting disk 50 comprises numbers ranging from 0 to 9. When the number 0 is used for forming the count, the second counting disk 50 is at the preset position, and the activator tooth 543 meshes with the transmission gear 80, so that the transmission gear 80 causes the third counting disk 70 to rotate once. For the counting mechanism 100 that displays the number of the remaining number of atomizations or actuations, when the number on the second counting disk 50 gradually decreases from 9 to 0, the ten digit is already gradually decreased, and a number tens unit reduction needs to be performed on the third counting disk 70, so that the ten digit is reduced or eliminated to gradually decrease the count.

For example, when the remaining number of times changes from 30 to 29, 0 on the second counting disk 50 is used for counting, the second counting disk 50 is located at the preset position, the second counting disk 50 is reduced from 0 to 9, and at the same time, the third counting disk 70 is reduced from 3 to 2, that is, the ones digit and the tens digit are both reduced in value by one. For another example, when the remaining number of actuations or aerosolizations changes from 29 to 28, the second counting disk 50 is not at the preset position, only the ones digit on the second counting disk 50 is reduced from 9 to 8, and the tens digit on the third counting disk 70 does not change.

It can be understood that, in some other embodiments, the counting mechanism 100 is configured to display a number of times that the inhalation device has been used, and the numbers on the second counting disk 50 include numbers ranging from 0 to 9. When the number 9 is used for forming the count, the second counting disk 50 is at the preset position, and the activator tooth 543 meshes with the transmission gear 80, so that the transmission gear 80 causes the third counting disk 70 to rotate once. The counting mechanism 100 displays the number of times that the inhalation device has been used, when the number on the second counting disk 50 gradually increases from 0 to 9, the ten digit is already gradually increased. A change in the tens digit value needs to be performed on the third counting disk 70 to produce an increase in the value of the tens digit once, to gradually increase the count.

In some embodiments, a first limiting member 60 is positioned on the shell 10. The second counting disk 50 is limited and stopped by the first limiting member 60 once each time number changes. In this way, rotation of the second counting disk 50 is limited and stopped by the first limiting member 60 once each time rotation occurs, to prevent the second counting disk 50 from rotating excessively, ensuring accuracy of each number change.

In some embodiments, at least some of the tooth grooves of the transmission teeth 541 form first limiting grooves 542, and a first limiting portion abuts against and is limited by any of the plurality of first limiting grooves 542 in a rotation direction of the second counting disk 50. In this way, each time the second counting disk 50 rotates, the first limiting member 60 abuts against and is limited by a different first limiting groove 542. Through cooperation between the first limiting member 60 and the first limiting groove 542, rotation of the second counting disk 50 is limited and stopped each time the number on the second counting disk 50 changes, thereby preventing the second counting disk 50 from rotating excessively and ensuring accuracy of each number change.

Referring to FIG. 6, FIG. 8, and FIG. 9, in some embodiments, a second limiting member 90 is positioned on the shell 10, and the third counting disk 70 is limited and stopped by the second limiting member 90 each time the number on the third counting disk 70 changes. In this way, the third counting disk 70 is limited and stopped by the second limiting member 90 each time the number on the third counting disk70 changes, to prevent the third counting disk 70 from rotating excessively, ensuring accuracy of each number change.

Further, the third counting disk 70 comprises a third disk body 72 and a gear portion 74. The third disk body 72 is provided with a plurality of numbers on a surface. The gear portion 74 is positioned on the third disk body 72 and is in mesh transmission with the transmission gear 80, so that through the mesh transmission between the gear portion 74 and the transmission gear 80, the third counting disk 70 is driven to rotate when the transmission gear 80 rotates. At least some of the tooth grooves in the gear portion 74 are configured as second limiting grooves 741, and the second limiting grooves 741 are provided corresponding to the plurality of numbers on the third disk body 72. In other words, at least some of the plurality of tooth grooves on an outer periphery of the gear portion 74 can be used as the second limiting grooves 741 to abut against a second limiting portion.

During rotation of the third counting disk 70, the second limiting member 90 abuts against and is limited by any of the plurality of limiting grooves in a rotation direction of the third counting disk 70. In this way, each time the third counting disk 70 rotates, the second limiting member 90 abuts against and is limited by a different second limiting groove 741. Through cooperation between the second limiting member 90 and the second limiting groove 741, the third counting disk 70 is limited and stopped each time the number changes, thereby preventing the third counting disk 70 from rotating excessively.

For the gear portion 74, a plurality of tooth grooves are distributed on the outer periphery of the gear portion 74. Some of the plurality of tooth grooves are configured to mesh with the transmission gear 80, and one of another part of the plurality of tooth grooves are configured for the second limiting member 90 to abut against. In this way, the second limiting portion is staggered from the transmission gear 80. For example, the second limiting portion and the transmission gear 80 are positioned on two opposite sides of the gear portion 74, and the second limiting portion does not affect normal meshing between the transmission gear 80 and the gear portion 74, and can limit and stop during each number change.

It can be understood that, the "number" in the foregoing implementations can be in a numeric form of any language, for example, any one of Arabic numbers, Roman numbers, English numbers, Chinese numbers, or the like. A carry rule form of counting can also be any existing counting rule form, such as decimal, binary, or duodecimal. In some other implementations, the "number" can alternatively be a self-defined mark symbol. The counting rule can alternatively be a user-defined counting rule.

In an embodiment, an inhalation device is further provided, including an atomization assembly and the counting mechanism 100 according to any one of the foregoing embodiments. The atomization assembly is configured to atomize an aerosol-forming material, and the count changes once each time the atomization assembly performs atomization. Specifically, the count of the counting assembly 20 changes one increment each time the atomization assembly performs and atomization or actuation. In this way, the counting mechanism 100 displays the number of times that the atomization assembly has been used or displays the remaining number of uses remaining, so that a user can accurately learn the number of times that the atomization assembly has been used. This is convenient to replace a new tank or inhalation device in a timely manner.

The various technical features in the foregoing embodiments can be randomly combined. For concise description, not all possible combinations of the various technical features in the above embodiments are described. However, provided that combinations of these technical features do not conflict with each other, the combinations of the various technical features are considered as falling within the scope of this specification.

The foregoing embodiments only describe several implementations of this application. The descriptions are specific and detailed, but should not be understood as limitations of the patent scope of this application. It should be noted that for a person of ordinary skill in the art, several transformations and improvements can be made without departing from the idea of this application. These transformations and improvements belong to the protection scope of this application. Therefore, the protection scope of this application shall be subject to the appended claims.

## Claims

1. A counting mechanism configured to count the number of times of atomization of an inhalation device, comprising:
a shell comprising a housing and a magnifying lens, wherein the housing has an observation window, and the magnifying lens is mounted at the observation window; and
a counting assembly positioned inside the housing and configured to form different dosage counts at the observation window.

2. The counting mechanism of claim 1, wherein the magnification of the magnifying lens is 2x to 3x.

3. The counting mechanism of claim 1, wherein:
the counting assembly comprises a first driving disk and a first counting disk rotatably positioned on the housing, and
each time the first driving disk rotates, the first counting disk rotates along with the rotation of the first driving disk and the count changes once.

4. The counting mechanism of claim 3, wherein when the first counting disk rotates to a rotation limit position, the first counting disk abuts against and is limited by the housing.

5. The counting mechanism of claim 4, wherein a reminder mark is provided on the first counting disk, and when the first counting disk rotates to the limit position, a reminder mark rotates to the observation window.

6. The counting mechanism of claim 1, wherein the counting assembly comprises a second driving disk, a second counting disk, and a third counting disk that are all rotatably positioned on the housing,
wherein the second counting disk has a geared connection with the second driving disk, and the third counting disk has a geared connection with the second counting disk,
wherein each time the second driving disk rotates, the second counting disk rotates along with the rotation of the second driving disk, and a dosage count number on the second counting disk changes once; and
wherein a dosage count number on the third counting disk changes once when the second counting disk rotates to a preset position, and the dosage count number on the second counting disk and the dosage count number on the third counting disk together form a total dosage count.

7. The counting mechanism of claim 6, wherein a receiving space is provided on the second counting disk, the third counting disk is eccentrically positioned in the receiving space, and any number on the third counting disk and any number on the second counting disk can be rotated to be adjacent and to form a total dosage count together.

8. The counting mechanism of claim 6 or 7, wherein the second counting disk comprises
a second disk body having a plurality of numbers on a surface thereof, and
a ring gear positioned on the second disk body and having a geared connection with the second driving disk.

9. The counting mechanism of claim 8, wherein transmission teeth are circumferentially positioned on an inner periphery of the ring gear, and the second driving disk is positioned in the ring gear and is in mesh transmission with the transmission teeth of the ring gear.

10. The counting mechanism of claim 8, further comprising a transmission gear having a geared connection between the ring gear and the third counting disk, and the third counting disk is driven by the transmission gear to rotate and undergo a number change only when the second counting disk rotates to the preset position.

11. The counting mechanism of claim 10, wherein an activator tooth is positioned on an outer periphery of the ring gear, and the activator tooth is intermittently meshed with the transmission gear only when the second counting disk rotates to the preset position.

12. An inhalation device comprising an atomization assembly and the counting mechanism of any one of claims 1 to 11, wherein each time the atomization assembly performs an atomization, the dosage count changes once.
